# EUROPÄISCHE PATENTSCHRIFT

(11) **EP 2 723 314 B1**
(45) Veröffentlichungstag und Bekanntmachung des Hinweises auf die Patenterteilung: **02.10.2019**
(21) Anmeldenummer: 12732571.0
(22) Anmeldetag: 15.06.2012
(51) Int. Cl.: C08F 2/26, A61K 8/81, A61Q 5/00, A61Q 19/10, C08F 2/22, C08F 220/18, C08F 220/26, C11D 3/37

(54) **NEUES VERDICKENDES POLYMER II**
NOVEL THICKENING POLYMER II
NOUVEAU POLYMÈRE ÉPAISSISSANT II

(30) Priorität: 27.06.2011 DE 102011078087
(43) Veröffentlichungstag der Anmeldung: 30.04.2014
(73) Patentinhaber: Beiersdorf AG, 20253 Hamburg (DE)
(72) Erfinder: MERTENS, Sascha, 21465 Reinbek (DE)
(74) Vertreter: Wihstutz, Kolja
(86) Internationale Anmeldenummer: PCT/EP2012/061507
(87) Internationale Veröffentlichungsnummer: WO 2013/017328

(56) Entgegenhaltungen:
- WO-A1-03/061615
- WO-A1-03/062288
- WO-A1-2011/151091

## Beschreibung

Die Erfindung betrifft ein neuartiges verdickendes Polymer mit besonderen Eigenschaften, das frei von assoziativen Monomeren ist bzw. dessen Kette keine solchen assoziativen Monomere enthält. Alle Mengenangaben sind- sofern nicht anders angegeben - Massenprozente bezogen auf die Gesamtmasse der Zubereitung.

Assoziative Monomere im Sinne der vorliegenden Schrift sind Monomere vom Typ U-S-L, wobei U eine ethylenisch ungesättigte Gruppe wie Methacrylat-, Acrylat-, Allyl- oder Vinylgruppen enthält, S ein Spacer aus einer Polyoxyalkylenkette mit 2 bis 300 Alkylenoxyeinheiten wie Polyethylenglykol, Polypropylenglykol oder Polybutylenglykol ist und L eine Hydrophobe Gruppe wie eine Alkyl- und/oder Arylgruppe ist, die wenigstens 4, bevorzugt wenigstens 8 Kohlenstoffatome aufweist, beispielsweise eine Laurylgruppe.

tan δ -Wert im Sinne der vorliegenden Schrift bedeutet der Quotient aus dem Verlustmodul und dem Speichermodul, jeweils gemessen bei 40°C. Dabei kommt es auf das verwendete Messgerät nicht an. Die in dieser Schrift angegebenen Werte wurden mit dem Malvern Gemini HR nano bestimmt. Allgemein dienen verdickende Polymere dazu, Wasser oder wasserhaltige Zubereitungen zu verdicken. Dabei lässt das Wasser die Polymere zu Gelen aufquellen, um so Viskosität und Fließverhalten zu beeinflussen.

Verdickende Polymere sind von Bedeutung bei der Herstellung von Körperreinigungsmitteln, Cremes, Reinigungsmitteln, Appreturen, Druckfarben, Lacken, Dispersionsfarben und anderen Beschichtungsstoffen, Klebstoffen, Papier, Lebensmitteln und so fort.

Aus der EP 1465932 B1 waren Alkali-quellbare und alkalilösliche assoziative Mehrzweck-Polymere, die das Polymerisationsprodukt eines Monomergemisches aus
(a) mindestens einem säurehaltigen Vinyl-Monomer,
(b) mindestens einem nichtionischem Vinyl-Monomer,
(c) einem ersten assoziativen Monomer, das eine erste hydrophobe Endgruppe hat,
(d) einem zweiten assoziativen Monomer mit einer zweiten hydrophoben Endgruppe, einem halbhydrophoben Monomer und aus einer Kombination davon und optional
(e) einem oder mehreren querverbindenen Monomeren oder Kettenüberträgern ist, bekannt.

Solche Polymere werden durch Emulsionspolymerisation hergestellt. Dabei wird zunächst die Monomermischung mit einem Tensid in Wasser in Form von Mizellen emulgiert oder suspendiert. In den eigentlichen Reaktor wird der Initiator in wässriger Lösung oder Suspension vorgelegt und die Monomersuspension langsam zugegeben. Die Geschwindigkeit der Monomerzugabe wird so gesteuert, dass es nicht zu einem erheblichen Anstieg der Reaktionstemperatur durch den Trommsdorff-Effekt (Geleffekt) kommt. Der Geleffekt tritt bei einem zu starken Anstieg des Umsatzes auf und führt dazu, dass die Polymerisationsgeschwindigkeit durch Hinderung der Diffusion der Polymerradikale ansteigt und sich die Polymerisation selbst beschleunigt. Dies versucht man aus Gründen der Sicherheit und zur Erhalt einer engen Molmassenverteilung zu verhindern. Tritt der Geleffekt auf, so erhält man eine bimodale Molmassenverteilung.

EP 1467698 A1 (WO 2003/061615 A1) offenbart Rheologie-modifizierende Polymere, die auch eine haarfestigende Wirkung haben. Dies können HASE-Polymere (hydrophobically modified alkali-swellable and alkali-soluble emulsion polymers) oder ASAP-Polymere (acidic/anionic alkali-swellable and/or alkali-soluble associative polymers) sein. Beide Polymere enthalten assoziative Monomere. Alle Beispielpolymere enthalten wenigstens ein assoziatives Monomer in Mengen ≥ 2,47 Gew. %.
Ebenfalls offenbart wird auch ein Syntheseverfahren für HASE-Polymere und ASAP-Polymere. Es wird beschrieben, dass eine Monomeremulsion zubereitet wird. Angaben zum Zeitpunkt, wann das quervernetzende Monomer zugegeben wird, fehlen.

Im Dokument WO 2011/151091 A1 wird die Synthese und die Verwendung eines Polymers, das aus wenigstens einem sauren Vinylmonomer oder dessen Salz, wenigstens einem nichtionischen Vinylmonomer, besonders bevorzugt einem hydrophoben nichtionischen Vinylmonomer, wenigstens einem Monomer, enthaltend eine ungesättigte Endgruppe und einen Polyoxyalkylenteil, wenigstens einem quervernetzenden Monomer, optional einem Schutzkolloid hergestellt wird, offenbart. Die Polymerisation wird dabei so gesteuert, dass zumindest zeitweise der Geleffekt auftritt, dadurch erreicht, dass die Zugabe der Monomeren (Dosierzeit),während 40 Minuten, besonders bevorzugt während 30 Minuten erfolgt. Es werden dieselben Arten von Monomeren eingesetzt, wie in der vorliegenden Erfindung. Jedoch fehlt keine Offenbarung, dass die Zugabe des quervernetzenden Monomers frühestens 10 Minuten nach Reaktionsstart erfolgen darf.

Weiterer Stand der Technik findet sich in EP 1272159, WO03062288, WO03062288, WO2007090759, US6242531.

Wünschenswert sind verdickende Polymere, mit denen es möglich ist, wässrige Zubereitungen so zu verdicken, dass möglichst klare Gele entstehen. Insbesondere war es wünschenswert, Körpereinigungsprodukte mit Trübungswerten unter 20 NTU bereitzustellen.

Weiterhin wünschenswert sind verdickende Polymere, mit denen in wässriger Lösung möglichst geringe tan δ-Werte (in Worten: Tangens delta) erreicht werden können. Insbesondere war es wünschenswert, Körpereinigungsprodukte mit tan δ-Werten unter 0,5 (in Worten: Tangens delta) bereitzustellen.

Ebenfalls wünschenswert sind verdickende Polymere mit einer deutlich erweiterten Lagerstabilität. Insbesondere war es wünschenswert, verdickte Körpereinigungsprodukte mit einer guten Lagerstabilität bei Temperaturen oberhalb von 40°C bereitzustellen, besonders solche, die sich nicht eintrüben.

Ebenfalls wünschenswert sind verdickende Polymere mit denen transparente kosmetische Reinigungsprodukte hergestellt werden können, die, nahezu gleichmäßig verteilt, sichtbare Partikel oder Blasen enthalten. Diese sollen auch nach längeren Standzeiten bei unterschiedlichen Temperaturen (0° - 40°C) in ihrer Position nahezu unverändert bleiben und auch für Zubereitungen mit pH-Werten unterhalb von 6,4 geeignet sein und dabei eine anwendungsgerechte Viskosität und eine zur Stabilisierung der Partikel notwendige Viskoelastizität gewährleisten. Dies ist wünschenswert, damit die Konservierungsmittel Benzoesäure und / oder Salizylsäure und deren Salze eingesetzt werden können. Diese organischen Säuren entfalten eine hinreichende antimikrobielle Wirkung aber erst in einem pH-Bereich < 6,4; das bedeutet, dass bislang Formulierungen der gewünschten Art mit weniger bevorzugten Konservierungsmitteln stabilisiert werden mussten.

Ebenfalls wünschenswert sind verdickende Polymere, die kompatibel mit anionischen (bis 10%), amphoteren (bis 10%) und nichtionischen Tensiden bei Gesamttensideinsatzkonzentrationen bis zu 20% sind.

Ebenfalls wünschenswert sind verdickende Polymere, die kompatibel mit kationischen Polymeren (bis 0,5%) und unempfindlich gegen Elektrolyte (0,1-4%, bevorzugt bis 3%, besonders bevorzugt bis 2%) sind.

Ebenfalls wünschenswert sind verdickende Polymere, die keinen negativen Einfluss auf Schaumentwicklung und -sensorik haben, einfach und stabil zu lagern und einfach einzuarbeiten sind.

Dem Stand der Technik fehlte es an verdickenden Polymeren, die die Herstellung klarer Gele bei gleichzeitig geringen tan δ-Werten (in Worten: Tangens delta) ermöglichen, wobei nach Möglichkeit zugleich zusätzlich das Gel einen pH-Werten unterhalb von 6,4, eine Fließgrenze und eine Kompatibilität mit anionischen (bis 10%), amphoteren (bis 10%) und nichtionischen Tensiden bei Gesamttensideinsatzkonzentrationen bis zu 20% und eine Unempfindlichkeit gegen Elektrolyte (0,1-4%) erreicht wird.

Überraschend und für den Fachmann nicht vorhersehbar hat sich nun herausgestellt, dass ein Polymer erhältlich durch radikalische Emulsionspolymerisation von
(A) wenigstens einem sauren Vinylmonomer oder dessen Salz,
(B) wenigstens einem nichtionischen Vinylmonomer, besonders bevorzugt einem hydrophoben nichtionischen Vinylmonomer,
(C) wenigstens einem Monomer, enthaltend eine ungesättigte Endgruppe und einen Polyoxyalkylenteil,
(D) wenigstens einem quervernetzenden Monomeren,
(E) optional einem Schutzkolloid
   dadurch gekennzeichnet, dass die Polymerisation so gesteuert wird, dass
(F) zumindest zeitweise der Geleffekt auftritt, dadurch erreicht, das die Monomerzugabe der Monomere (A), (B) und (C) (Dosierzeit) während 120 Minuten, bevorzugt 60 Minuten, besonders bevorzugt 40 Minuten, ganz besonders bevorzugt 30 Minuten erfolgt und wobei die Zugabe der Monomere konstant ist und gleichzeitig bei hoher Zugabegeschwindigkeit der Monomere erfolgt und
(G) die Zugabe des quervernetzenden Monomeren (D) frühestens 10 Minuten, bevorzugt frühestens 15 Minuten nach der ersten Zugabe der Monomere (A), (B) und (C) beginnt und dass
(H) assoziative Monomere fehlen oder höchstens eine Konzentration von 1 Gew.%, bevorzugt 0,1 Gew.% aufweisen,
den Mängeln des Standes der Technik abhilft.

Bei einer solchen Polymerisation unter Ausnutzung des Trommsdorff-Effektes,
d.h. bei konstanter Zugabe der Monomere und gleichzeitig hoher Zugabegeschwindigkeit der Monomere bildet sich ein Monomerenüberschuß, der zu einer Selbstbeschleunigung der Polymerisation (Trommsdorff-Effekt) führt. Das Ergebnis ist ein Anstieg der Molekulargewichte bei gleichzeitig vorteilhafter Morphologie der Polymere. Dabei ist es von Vorteil, wenn die Temperatur während der Polymerisation zwischen 70 und 90°C, bevorzugt 80 und 90°C gehalten wird. Weiter von Vorteil ist es, wenn die Initiatorzugabe sowohl vor Beginn der Dosierzeit, als auch nach Zugabe des quervernetzenden Monomers erfolgt. Dabei ist es besonders bevorzugt, wenn (H) assoziative Monomere fehlen oder höchstens eine Konzentration von 0,1 Gew.% aufweisen. Besonders bevorzugt ist es, wenn das saure Vinylmonomer (A) gewählt wird unter Vinylmonomeren mit Carboxylgruppen, besonders bevorzugt Acrylsäure oder Methacrylsäure oder deren Alkali-, Erdalkali-, Ammonium oder Alkylammoniumsalzen. Besonders bevorzugt ist es, wenn das nichtionische Vinylmonomer (B) gewählt wird aus der Gruppe der C1-C22-Alkylacrylate und der C1-C22-Alkymethacrylate sowie deren Mischungen. Dadurch werden gute Fließeigenschaften und damit ein vorteilhaftes rheologisches Profil erreicht. Besonders bevorzugt ist es, wenn das Monomer (C), enthaltend eine ungesättigte Endgruppe und einen Polyoxyalkylenteil gewählt wird unter Vinylpolyalkylenglykolen oder polymerisierbaren Tensiden oder deren Mischungen, besonders bevorzugt gewählt wird unter Vinylpolyalkylenglykolen oder polymerisierbaren Tensiden oder deren Mischungen, bevorzugt gewählt wird unter ethoxyliertem und propoxyliertem 1,4-Butandiolvinylether mit 30 Ethylenoxy- und Propylenoxyeinheiten, Allylpolyethylenglykolether mit 30 Ethylenoxyeinheiten, Allylpolyethylenglykolether mit 20 Ethylenoxyeinheiten, Vinylpolyethylenglykolether mit 20 Ethylenoxyeinheiten, CH2=CHCH2O[(CH2CH2O)n(CH2(CH3)CHO)]mCH3 mit m+n = 5 bis 100 und n/m = 1, Polyalkylenglykolallylbutylether mit 25 Ethylenoxy- und 8 Propylenoxyeinheiten, ganz besonders bevorzugt werden eingesetzt Emulsogen R307 (EO/PO 30 1,4-Butandiolvinylether (EO/PO 30 mol), Clariant), Emulsogen RAL307 (Allylpolyalkylenglykolether (EO 30 mol), Clariant), Polyglykol A11/1800 (Allylpolyalkylenglykolether (EO 20 mol, PO 20 mol), Clariant), Polyglykol R1100 (Vinylpolyalkylenglykolether (EO 20 mol), Clariant), Pluriol A111 R (Allylalkoholalkoxylate, BASF) oder Polyglykol AB25-8 (Polyalkylenglykolallylbutylether (EO 25 mol, PO 8 mol), Clariant). Besonders bewährt hat sich Polyglykol A11/1800, was daher außergewöhnlich bevorzugt ist. Besonders bevorzugt ist es, wenn das quervernetzende Monomer (D) gewählt wird unter Polyol(meth)acrylaten mit wenigstens zwei (Meth)acrylatgruppen und den Mischestern von Polyolen mit Acrylsäure und/oder Methacrylsäure. Weiter besonders bevorzugt ist es, wenn die Monomere (A) in Gehalten von 10 bis 75%, bevorzugt 20 bis 60%, besonders bevorzugt 28 bis 52%,, ganz besonders bevorzugt 32 bis 52%, (B) in Gehalten von 10 bis 90%, bevorzugt 30 bis 80%, besonders bevorzugt 40 bis 62%, ganz besonders bevorzugt 40 bis 60%, (C) in Gehalten von 0,5 bis 40%, bevorzugt 1 bis 10%, besonders bevorzugt 2 bis 6%, (D) in Gehalten von bis zu 1%, bevorzugt 0,05 bis 0,5%, besonders bevorzugt 0,1 bis 0,3% vorliegen. Ganz besonders bevorzugt ist es, wenn die Monomere (A):(B) in Massenverhältnissen von 1:2,2 bis 1,5:1, bevorzugt 1:1,6 bis 1,3:1 vorliegen. Ganz besonders bevorzugt ist es, wenn die Zugabe des quervernetzenden Monomeren frühestens nach 10 Minuten, besonders bevorzugt nach 15 Minuten beginnt und entweder sofort endet oder bis zum Ende der Dosierzeit der Monomere andauert. Ganz besonders bevorzugt ist es, wenn das Polymer unter Beispiel 7, 8 oder 12 gewählt wird. Die Erfindung umfasst auch eine verdickte Zubereitung enthaltend ein Polymer nach einem der vorangehenden Patentansprüche. Besonders bevorzugt ist es, wenn die Einsatzkonzentration des Polymers zur Verdickung wässriger, bevorzugt tensidhaltiger wässriger Zubereitungen 0,5 bis 5%, bevorzugt 1 bis 4%, besonders bevorzugt bis 3% beträgt. Dadurch wird die Zubereitung bei pH-Werten <6,5 transparent und weist eine Viskosität von mindestens 2000 mPa.s auf, was es ermöglicht, dass sich zugesetzte Schwebestoffe nicht absetzen, wobei die Schwebestoffe Perlen, Pigmente oder Luftblasen sein können. Besonders bevorzugt ist es, wenn die erfindungsgemäßen Zubereitungen ein Gesundheitsprodukt, Reinigungsprodukt, Haushaltsprodukt, Duschgel, Anstrichmittel, Tinten, Dispergiermittel, Antiabsetzmittel, Beton- und Zementzusatzmittel, Beschichtungen, Medizinprodukt, kosmetisches Produkt oder dermatologisches Produkt sind. Die Erfindung umfasst auch die Verwendung eines erfindungsgemäßen Polymers als Verdicker, Emulgator, Dispergiermittel oder Konsistenzgeber.

Vorteilhaft werden die Zubereitungen - sofern es sich um gelformige Zubereitungen mit Fließgrenze handelt - so ausgestaltet, dass sie eine Fließgrenze von 0,5-20 Pa aufweisen, bevorzugt 1-6 Pa.

Als Fließgrenze wird die kritische Schubspannung der Fließkurve angesehen. Sie kann erfindungsgemäß wie folgt ermittelt werden:
Gemessen wird die Fließkurve auf einem schubspannungsgesteuerten Rheometer bei 25°C ± 1 °C mit 20 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Vorteilhaft werden die Zubereitungen so ausgestaltet, dass sie einen tan δ (in Worten: Tangens delta) von 0,05-0,6 aufweisen, bevorzugt 0,1-0,5.

Unter tan δ (in Worten: Tangens delta) wird erfindungsgemäß der Quotient aus dem Verlustmodul und dem Speichermodul verstanden. Der tan δ wird wie folgt ermittelt: Gemessen werden Verlust- und Speichermodul durch einen dynamischen Frequenztestlauf einem schubspannungsgesteuerten Rheometer bei 40°C ± 1 °C mit 20 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan δ (in Worten: Tangens delta) im Frequenzbereich zwischen 0,05 rad/s und 3,0 rad/s angegeben, bevorzugt zwischen 0,08 rad/s und 1,0 rad/s.

Vorteilhaft werden die Zubereitungen so ausgestaltet, dass Sie bei pH < 6.2 einen Trübungswert von NTU (Nephelometric Turbidity Unit) < 20 aufweisen. Der Trübungswert wird gemessen mit einem Trübungsmessgerät, wobei destilliertes Wasser mit einem Wert von NTU=0 als Standard gilt.

Erfindungsgemäß ist es auch, wenn nicht Wasser, sondern andere polare Flüssigkeiten durch erfindungsgemäße Polymere verdickt werden. Insbesondere können Alkohole, Glykole, Polyole, Amine und organische Säuren wie etwa Acrylsäure sowie deren wässrige Lösungen verdickt werden.

Erfindungsgemäß ist auch die Verwendung erfindungsgemäßer Polymere als Emulgator. So können Cremes und Lotionen bereitgestellt werden.

Die Polymere eignen sich auch als Dispergier- und Antiabsetzmittel.

### Beispiele

### Synthese der Copolymere

Die nachfolgenden Beispiele sollen die vorliegende Erfindung verdeutlichen, ohne sie einzuschränken.

Die Herstellung der Beispiele erfolgt gemäß der nachstehend beschriebenen Methode, wobei Art und
Mengen der jeweils als Ausgangskomponenten eingesetzten Monomere in der Tabelle 2 zusammengefasst wiedergegeben sind. Teile und Prozente beziehen sich auf das Gewicht.

### Beispiel 1:

Monomerphase
Methacrylsäure 37,000 Teile
Ethylacrylat 57,400 Teile
Polyglykol A11/1800 5,400 Teile
Vernetzerphase
Trimethylolpropantriacrylat 0,300 Teile
Wasserphase
Wasser 33,060 Teile
Natriumlaurylsulfat 0,984 Teile
Reaktionsgefäß
Wasser 173,146 Teile
Natriumlaurylsulfat 0,300 Teile
Initiatorphase A
Wasser 1,911 Teile
Ammoniumpersulfat 0,069 Teile
Initiatorphase B
Wasser 2,909 Teile
Ammoniumpersulfat 0,021 Teile

Im Reaktionsgefäß, das mit Rührer, Rückflusskühler, Stickstoffzuleitung, Dosiervorrichtung und Innenthermometer ausgestattet ist, werden 173,146 Teile Wasser und 0,300 Teile Natriumlaurylsulfat vorgelegt. Die Mischung wird unter Rühren und Stickstoffatmosphäre auf 82°C erwärmt.
Das Monomerengemisch wird in einem zweiten Rührgefäß hergestellt, das mit Rührer und Stickstoffzuleitung ausgestattet ist. Dazu wird die Monomerphase mit 37,000 Teilen Methacrylsäure, 57,400 Teilen Ethylacrylat und 5,400 Teilen Polyglykol A11/1800 vorlegt und in diese unter Rühren und Stickstoffatmosphäre die Wasserphase mit 33,060 Teilen Wasser und 0,984 Teilen Natriumlaurylsulfat gemischt.

Sobald im Reaktionsgefäß eine Temperatur von 82°C erreicht ist, wird eine Initiatorphase A, bestehend aus 0,069 Teilen Ammoniumpersulfat und 1,911 Teilen Wasser zugegeben und das Monomerengemisch bei 85-88°C während 30 Minuten gleichmäßig zudosiert. 20 Minuten nach Beginn der Monomerdosierung wird die Vernetzerphase komplett in die Monomerphase gegeben. Nach Abschluss der Dosierung wird eine Initiatorphase B, bestehend aus 0,021 Teilen Ammoniumpersulfat und 2,909 Teilen Wasser zugegeben und anschließend die Reaktionsmischung noch 4 Stunden bei 90°C nachpolymerisiert, bevor auf <40°C abgekühlt wird.

### Beispiele 2-13:

Die nachfolgenden Beispiele (Tabelle 1) sind analog zu Beispiel 1 hergestellt. Das in der Tabelle angegebene Vernetzungsverfahren beschreibt die Art und den Zeitpunkt der Zugabe des Vernetzers. Der Buchstabe "R" bezieht sich auf eine Zugabe des Vernetzers in das Reaktionsgefäß, während der Buchstabe "M" eine Zugabe des Vernetzers in die Monomerphase beschreibt. Die Zahlen geben den Zeitpunkt der Zugabe des Vernetzers in Minuten nach Reaktionsbeginn (Start der Monomerdosierung) an. Das Beispiel 13 wurde wie Beispiel 1 hergestellt, jedoch wurde der Vernetzer nicht zu einem späteren Zeitpunkt als einzelne Vernetzerphase zugegeben, sondern mit den anderen Monomeren in der Monomerphase eingewogen und mit der Monomerphase während 30 Minuten zudosiert.

### Abkürzungen

MAA Methacrylsäure
EA Ethylacrylat
A11/1800 Polyglykol A11/1800 (Clariant)
Allylpolyalkylenglykolether (EO 20 mol, PO 20 mol)
TMPTA Trimethylolpropantriacrylat.

**Tabelle 1: Beispiele 1-13**

| **Polymer** | **1** | **2** | **3** | **4** | **5** | **6** | **7** | **8** | **9** | **10** | **11** | **12** | **13*** |
|---|---|---|---|---|---|---|---|---|---|---|---|---|---|
| MAA | 37 | 37 | 37 | 37 | 45,4 | 45,4 | 45,4 | 45,4 | 45,4 | 50 | 45,4 | 45,4 | 37 |
| EA | 57,4 | 57,4 | 57,4 | 57,25 | 49 | 49 | 49,05 | 49 | 49 | 44,4 | 49 | 49,05 | 57,4 |
| A11/1800 | 5,4 | 5,4 | 5,4 | 5,4 | 5,4 | 5,4 | 5,4 | 5,4 | 5,4 | 5,4 | 5,4 | 5,4 | 5,4 |
| TMPTA | 0,3 | 0,3 | 0,3 | 0,45 | 0,3 | 0,3 | 0,15 | 0,2 | 0,2 | 0,2 | 0,2 | 0,15 | 0,30 |
| | | | | | | | | | | | | | |
| Vernetzungsverfahren | M20 | R30 | M25 | R30 | R20 | M20 | M20 | M20 | M15 | M20 | R20 | R20 | - |

| | | | | | | | | | | | | | |
|---|---|---|---|---|---|---|---|---|---|---|---|---|---|
| * Vergleichsbeispiel, nicht erfindungsgemäß. | | | | | | | | | | | | | |

**Tabelle 2: Ergebnisse Polymeremulsionen**

| Polymer | pH-Wert | Partikelgröße* in nm |
|---|---|---|
| 1 | 2,55 | 86 |
| 2 | 2,53 | 87 |
| 3 | 2,49 | 84 |
| 4 | 2,63 | 90 |
| 5 | 2,51 | 99 |
| 6 | 2,55 | 99 |
| 7 | 2,49 | 95 |
| 8 | 2,59 | 94 |
| 9 | 2,63 | 93 |
| 10 | 2,53 | 104 |
| 11 | 2,53 | 96 |
| 12 | 2,50 | 93 |
| 13 | 2,50 | 89 |

| | | |
|---|---|---|
| *Die Partikelgröße in der Polymeremulsion wird mit Dynamischer Lichtstreuung (DLS) in einer verdünnten wässrigen Probe der Emulsion bei 25°C ± 1 °C ermittelt. | | |

Beispiel 14:

Alle Mengenangaben, Anteile und Prozentanteile sind, soweit nicht anders angegeben, auf das Gewicht und die Gesamtmenge bzw. auf das Gesamtgewicht der Zubereitung bezogen. Das erfindungsgemäße Polymer wird mit einem Teil der Wasserphase verdünnt und unter Rühren zur Tensidphase gegeben. Anschließend werden die weiteren Rezepturbestandteile bis auf Natronlauge, Zitronensäure und die Schwebekörper unter Rühren zugegeben. Nach erfolgter pH-Einstellung auf pH 5 werden die Schwebekörper in die fertige Gelgrundlage unter möglichst geringer Scherung eingerührt.

**Tabelle 3: Zubereitung Duschgel**

| | |
|---|---|
| Natriumlaurethsulfat | 9,50 |
| Cocoamidopropylbetain | 3,00 |
| Polymer | 2,25 |
| PEG-40 hydriertes Rizinusöl | 0,80 |
| PEG-7 Glyceryl Cocoat | 1,00 |
| Natriumbenzoat | 0,45 |
| Natriumsalicylat | 0,40 |
| Unispheres UEA-509 | 0,15 |
| Natronlauge | ad pH 6,0 |
| Zitronensäure | ad pH 5,0 |
| Parfum | 0,5. |
| Wasser | ad 100 |

**Tabelle 4: Ergebnisse Zubereitung Duschgel**

| Polymer im Duschgel | tan δ | Viskosität in mPas | Trübung in NTU |
|---|---|---|---|
| 1 | 0,24 | 3335 | 23 |
| 2 | 0,39 | 3057 | 7 |
| 3 | 1,00 | 3001 | 14 |
| 4 | 1,22 | 2761 | 10 |
| 5 | 0,12 | 3413 | 25 |
| 6 | 0,17 | 3021 | 26 |
| 7 | 0,24 | 2906 | 14 |
| 8 | 0,17 | 3525 | 19 |
| 9 | 0,11 | 4169 | 25 |
| 10 | 0,22 | 3089 | 23 |
| 11 | 0,12 | 2903 | 22 |
| 12 | 0,18 | 2825 | 18 |
| 13 | 0,07 | 5785 | 80 |

Die Viskosität der Zubereitungen wird auf einem Rheometer bei 25°C ± 1 °C mit 40 mm Kegel/Platte Geometrie (1° Kegelwinkel) bei einem Spalt von 0,03 mm gemessen, wobei strukturschonend befüllt wird. Es wird eine geeignete konstante Scherratenzeitrampe von 0,1 - 1000 s⁻¹ vorgegeben und vor dem Test eine entsprechende Strukturerholungszeit eingehalten. Die Viskosität wird bei einer Scherrate von 10 s⁻¹ angegeben.

Die Zubereitung 13, hergestellt mit dem Polymer nach Beispiel 13, weist eine zu hohe Viskosität und eine unerwünscht starke Trübung auf. Außerdem hat es eine zu hohe Fließgrenze, die sich in einem sehr niedrigen tan δ zeigt. Die hohe Fließgrenze führt dazu, dass die Zubereitung nicht mehr fließfähig ist und damit nicht von alleine aus einer Flasche fließen kann. Die Zubereitungen 1-12 haben diesen Nachteil nicht.

### Beispiel 15:

Bestimmte Polymere wie das Carbopol® AQUA SF-2 (Lubrizol) zeigen bei höheren Temperaturen eine ansteigende Trübung. Dies ist insbesondere in Ländern mit höheren Tagestemperaturen oder allgemein in den Sommermonaten unerwünscht. Und kann vom Verbraucher als mangelnde Produktqualität ausgelegt werden. Es wurde eine Zubereitung wie in Tabelle 3 gezeigt mit Carbopol® AQUA SF-2 und dem Polymer aus Beispiel 8 hergestellt und verglichen.

**Tabelle 5: Trübungswerte im Vergleich zu AQUA SF-2**

| | 22°C | 30°C | 35°C | 40°C | 50°C | 22°C | 22°C nach 72h | 22°C 1 Woche |
|---|---|---|---|---|---|---|---|---|
| Beispiel 8 | 18 | 19 | 18 | 18 | 18 | 19 | 18 | 18 |
| Aqua SF-2 | 24 | 24 | 26 | 37 | 140 | 31 | 27 | 26 |

Die erfindungsgemäßen Polymere weisen keine temperaturabhängige Trübung auf, während das Carbopol® AQUA SF-2 mit ansteigender Temperatur immer trüber wird.

## Patentansprüche

1. Polymer erhältlich durch radikalische Emulsionspolymerisation von
(A) wenigstens einem sauren Vinylmonomer oder dessen Salz,
(B) wenigstens einem nichtionischen Vinylmonomer, besonders bevorzugt einem hydrophoben nichtionischen Vinylmonomer,
(C) wenigstens einem Monomer, enthaltend eine ungesättigte Endgruppe und einen Polyoxyalkylenteil,
(D) wenigstens einem quervernetzenden Monomeren,
(E) optional einem Schutzkolloid,
**dadurch gekennzeichnet, dass** die Polymerisation so gesteuert wird, dass
(F) zumindest zeitweise der Geleffekt auftritt, dadurch erreicht, dass die Monomerzugabe der Monomere (A), (B) und (C) (Dosierzeit) während 120 Minuten, bevorzugt 60 Minuten, besonders bevorzugt 40 Minuten, ganz besonders bevorzugt 30 Minuten erfolgt und
wobei die Zugabe der Monomere konstant ist und gleichzeitig bei hoher Zugabegeschwindigkeit der Monomere erfolgt,
(G) die Zugabe des quervernetzenden Monomeren (D) frühestens 10 Minuten nach der ersten Zugabe der Monomere (A), (B) und (C) erfolgt,
und dass
(H) assoziative Monomere vom Typ U-S-L fehlen oder höchstens eine Konzentration von 1 Gew.%, bevorzugt 0,1 Gew.%, aufweisen und
wobei die assoziativen Monomere vom Typ U-S-L eine ethylenisch ungesättigte Gruppe wie Methacrylat-, Acrylat-, Allyl- oder Vinyl, entsprechend U, einen Spacer aus einer Polyoxyalkylenkette mit 2 bis 300 Alkylenoxyeinheiten wie Polyethylenglykol, Polypropylenglykol oder Polybutylenglykol, entsprechend S und eine hydrophobe Gruppe wie eine Alkyl- und/oder Arylgruppe, die wenigstens 4, bevorzugt wenigstens 8 Kohlenstoffatome aufweist, entsprechend L, enthalten.

2. Polymer nach einem der vorangehenden Patentansprüche, **dadurch gekennzeichnet, dass** das saure Vinylmonomer (A) gewählt wird unter Vinylmonomeren mit Carboxylgruppen, besonders bevorzugt Acrylsäure oder Methacrylsäure oder deren Alkali-, Erdalkali-, Ammonium oder Alkylammoniumsalzen, ganz besonders bevorzugt Methacrylsäure oder deren Alkali-, Erdalkali-, Ammonium oder Alkylammoniumsalze.

3. Polymer nach einem der vorangehenden Patentansprüche, **dadurch gekennzeichnet, dass** das nichtionische Vinylmonomer (B) gewählt wird aus der Gruppe der C1-C22-Alkyl-acrylate und der C1-C22-Alkyl-methacrylate sowie deren Mischungen.

4. Polymer nach einem der vorangehenden Patentansprüche, **dadurch gekennzeichnet, dass** das Monomer (C), enthaltend eine ungesättigte Endgruppe und einen Polyoxyalkylenteil, gewählt wird unter Vinylpolyalkylenglykolen oder polymerisierbaren Tensiden oder deren Mischungen, bevorzugt gewählt wird unter ethoxyliertem und propoxyliertem 1,4-Butandiolvinylether mit 30 Ethylenoxy- und Propylenoxyeinheiten, Allylpolyethylenglykolether mit 30 Ethylenoxyeinheiten, Allylpolyethylenglykolether mit 20 Ethylenoxyeinheiten, Vinylpolyethylenglykolether mit 20 Ethylenoxyeinheiten, CH₂=CHCH₂O[(CH₂CH₂O)ₙ(CH₂(CH₃)CHO)]ₘCH₃ mit m+n = 5 bis 100 und n/m = 1, Polyalkylenglykolallylbutylether mit 25 Ethylenoxy- und 8 Propylenoxyeinheiten.

5. Polymer nach einem der vorangehenden Patentansprüche, **dadurch gekennzeichnet, dass** das quervernetzende Monomer (D) gewählt wird unter Polyol(meth)acrylaten mit wenigstens zwei (Meth)acrylatgruppen und den Mischestern von Polyolen mit Acrylsäure und/oder Methacrylsäure.

6. Polymer nach einem der vorangehenden Patentansprüche, **dadurch gekennzeichnet, dass** die Monomere (A):(B) in Massenverhältnissen von 1:2,2 bis 1,5:1, bevorzugt 1:1,6 bis 1,3:1 vorliegen.

7. Polymer nach einem der vorangehenden Patentansprüche, **dadurch gekennzeichnet, dass** die Zugabe des quervernetzenden Monomeren frühestens nach 10 Minuten beginnt und entweder sofort endet oder bis zum Ende der Monomerzugabe der Monomere (A), (B) und (C) andauert.

8. Polymer nach einem der vorangehenden Patentansprüche, **dadurch gekennzeichnet, dass** es unter Beispiel 7, 8 oder 12 gewählt wird.

9. Verdickte Zubereitung, enthaltend ein Polymer nach einem der vorangehenden Patentansprüche.

10. Zubereitung nach Patentanspruch 9, **dadurch gekennzeichnet, dass** die Einsatzkonzentration des Polymers zur Verdickung wässriger, bevorzugt tensidhaltiger wässriger Zubereitungen 0,5 bis 5%, bevorzugt 1 bis 4% beträgt.

11. Zubereitung nach Patentanspruch 10, **dadurch gekennzeichnet, dass** die Zubereitung bei pH-Werten <6,5 transparent ist und eine Viskosität von mindestens 2000 mPa·s aufweist.

12. Zubereitung nach Patentanspruch 10 oder 11, **dadurch gekennzeichnet, dass** sie ein Gesundheitsprodukt, Reinigungsprodukt, Haushaltsprodukt, Duschgel, Anstrichmittel, Tinten, Dispergiermittel, Antiabsetzmittel, Beton- und Zementzusatzmittel, Beschichtungen, Medizinprodukt, kosmetisches Produkt oder dermatologisches Produkt ist.

13. Verwendung eines Polymers nach einem der Patentansprüche 1 bis 8 als Verdicker, Emulgator, Dispergiermittel oder Konsistenzgeber.

14. Verfahren zur Herstellung eines Polymers durch radikalische Emulsionspolymerisation **dadurch gekennzeichnet, dass** das Polymer erhältlich ist aus
(A) wenigstens einem sauren Vinylmonomer oder dessen Salz,
(B) wenigstens einem nichtionischen Vinylmonomer, besonders bevorzugt ein hydrophobes nichtionischen Vinylmonomer,
(C) wenigstens einem Monomer, enthaltend eine ungesättigte Endgruppe und einen Polyoxyalkylenteil,
(D) wenigstens einem quervernetzenden Monomer und
(E) optional einem Schutzkolloid,
wobei die Polymerisation so gesteuert wird, dass
(F) zumindest zeitweise ein Geleffekt auftritt, dadurch erreicht, dass die Monomerzugabe der Monomere (A), (B) und (C) während 120 Minuten, bevorzugt 60 Minuten, besonders bevorzugt 40 Minuten, ganz besonders bevorzugt 30 Minuten erfolgt,
wobei die Zugabe der Monomere konstant ist und gleichzeitig bei hoher Zugabegeschwindigkeit der Monomere erfolgt und
(G) die Zugabe des quervernetzenden Monomeren (D) frühestens 10 Minuten nach der ersten Zugabe der Monomere (A), (B) und (C) erfolgt und
(H) assoziative Monomere vom Typ U-S-L fehlen oder höchstens eine Konzentration von 1 Gew.%, bevorzugt 0,1 Gew.%, aufweisen und
wobei die assoziativen Monomere vom Typ U-S-L eine ethylenisch ungesättigte Gruppe wie Methacrylat-, Acrylat-, Allyl- oder Vinyl, entsprechend U, einen Spacer aus einer Polyoxyalkylenkette mit 2 bis 300 Alkylenoxyeinheiten wie Polyethylenglykol, Polypropylenglykol oder Polybutylenglykol, entsprechend S und eine hydrophobe Gruppe wie eine Alkyl- und/oder Arylgruppe, die wenigstens 4, bevorzugt wenigstens 8 Kohlenstoffatome aufweist, entsprechend L, enthalten.

15. Verfahren nach Patentanspruch 14 **dadurch gekennzeichnet, dass** die Zugabe des quervernetzenden Monomere frühestens nach 10 Minuten beginnt und entweder sofort endet oder bis zum Ende der Monomerzugabe der Monomere (A), (B) und (C) andauert.

## Claims

1. Polymer obtainable by radical emulsion polymerization of
(A) at least one acidic vinyl monomer or salt thereof,
(B) at least one nonionic vinyl monomer, particularly preferably a hydrophobic nonionic vinyl monomer,
(C) at least one monomer containing an unsaturated end group and a polyoxyalkylene moiety,
(D) at least one crosslinking monomer,
(E) optionally a protective colloid,
**characterized in that** the polymerization is controlled such that
(F)at least at times the gel effect arises, achieved by adding the monomers (A), (B) and (C) (dosing time) within 120 minutes, preferably 60 minutes, particularly preferably 40 minutes, very particularly preferably 30 minutes and
wherein the addition of the monomers is constant and takes place with simultaneously high rate of addition of the monomers,
(G) the crosslinking monomer (D) is added at the earliest 10 minutes after the first addition of the monomers (A), (B) and (C),
and **in that**
(H) associative monomers of the U-S-L type are missing or at most have a concentration of 1% by weight, preferably 0.1% by weight and
wherein the associative monomers of the U-S-L type contain an ethylenically unsaturated group such as methacrylate, acrylate, allyl or vinyl, corresponding to U, a spacer made of a polyoxyalkylene chain having 2 to 300 alkyleneoxy units such as polyethylene glycol, polypropylene glycol or polybutylene glycol, corresponding to S, and a hydrophobic group such as an alkyl and/or aryl group, which has at least 4, preferably at least 8, carbon atoms, corresponding to L.

2. Polymer according to one of the preceding claims, **characterized in that** the acidic vinyl monomer (A) is selected from vinyl monomers with carboxyl groups, particularly preferably acrylic acid or methacrylic acid or alkali metal, alkaline earth metal, ammonium or alkylammonium salts thereof, very particularly preferably methacrylic acid or alkali metal, alkaline earth metal, ammonium or alkylammonium salts thereof.

3. Polymer according to one of the preceding claims, **characterized in that** the nonionic vinyl monomer (B) is selected from the group of the C1-C22-alkyl acrylates and of the C1-C22-alkyl methacrylates, and mixtures thereof.

4. Polymer according to one of the preceding claims, **characterized in that** the monomer (C) containing an unsaturated end group and a polyoxyalkylene moiety is selected from vinylpolyalkylene glycols or polymerizable surfactants or mixtures thereof, is preferably selected from ethoxylated and propoxylated 1,4-butanediol vinyl ether with 30 ethyleneoxy and propyleneoxy units, allylpolyethylene glycol ether with 30 ethyleneoxy units, allylpolyethylene glycol ether with 20 ethyleneoxy units, vinylpolyeth-ylene glycol ether with 20 ethyleneoxy units, CH₂=CHCH₂O[(CH₂CH₂O)ₙ(CH₂(CH₃)CHO)]ₘCH₃ where m+n = 5 to 100 and n/m = 1, polyalkylene glycol allyl butyl ether with 25 ethyleneoxy and 8 propyleneoxy units.

5. Polymer according to one of the preceding claims, **characterized in that** the crosslinking monomer (D) is selected from polyol (meth)acrylates with at least two (meth)acrylate groups and the mixed esters of polyols with acrylic acid and/or methacrylic acid.

6. Polymer according to one of the preceding claims, **characterized in that** the monomers (A):(B) are present in mass ratios of from 1:2.2 to 1.5:1, preferably 1:1.6 to 1.3:1.

7. Polymer according to one of the preceding claims, **characterized in that** the addition of the crosslinking monomer starts at the earliest after 10 minutes and either ends immediately or lasts until the end of the monomer addition of the monomers (A), (B) and (C).

8. Polymer according to one of the preceding claims, **characterized in that** it is selected from example 7, 8 or 12.

9. Thickened preparation comprising a polymer according to one of the preceding claims.

10. Preparation according to Claim 9, **characterized in that** the use concentration of the polymer for thickening aqueous, preferably surfactant-containing aqueous, preparations is 0.5 to 5%, preferably 1 to 4%.

11. Preparartion according to Claim 10, **characterized in that** the preparation is transparent at pH values <6.5 and has a viscosity of at least 2000 mPa.s.

12. Preparation according to Claim 10 or 11, **characterized in that** it is a health product, cleaning product, household product, shower gel, paint, inks, dispersant, anti-settling agent, concrete and cement additive, coatings, medicinal product, cosmetic product or dermatological product.

13. Use of a polymer according to one of Claims 1 to 8 as thickener, emulsifier, dispersant or consistency regulator.

14. Process for producing a polymer by radical emulsion polymerization, **characterized in that** the polymer is obtainable from
(A) at least one acidic vinyl monomer or salt thereof,
(B) at least one nonionic vinyl monomer, particularly preferably a hydrophobic nonionic vinyl monomer,
(C) at least one monomer containing an unsaturated end group and a polyoxyalkylene moiety,
(D) at least one crosslinking monomer and
(E) optionally a protective colloid,
wherein the polymerization is controlled such that
(F)at least at times a gel effect arises, achieved by adding the monomers (A), (B) and (C) within 120 minutes, preferably 60 minutes, particularly preferably 40 minutes, very particularly preferably 30 minutes,
wherein the addition of the monomers is constant and takes place with simultaneously high rate of addition of the monomers and
(G) the crosslinking monomer (D) is added at the earliest 10 minutes after the first addition of the monomers (A), (B) and (C) and
(H) associative monomers of the U-S-L type are missing or at most have a concentration of 1% by weight, preferably 0.1% by weight and
wherein the associative monomers of the U-S-L type contain an ethylenically unsaturated group such as methacrylate, acrylate, allyl or vinyl, corresponding to U, a spacer made of a polyoxyalkylene chain having 2 to 300 alkyleneoxy units such as polyethylene glycol, polypropylene glycol or polybutylene glycol, corresponding to S, and a hydrophobic group such as an alkyl and/or aryl group, which has at least 4, preferably at least 8, carbon atoms, corresponding to L.

15. Process according to Claim 14, **characterized in that** the addition of the crosslinking monomer starts at the earliest after 10 minutes and either ends immediately or lasts until the end of the monomer addition of the monomers (A), (B) and (C).

## Revendications

1. Polymère, pouvant être obtenu par polymérisation en émulsion radicalaire de :
(A) au moins un monomère de vinyle acide ou son sel,
(B) au moins un monomère de vinyle non ionique, de manière particulièrement préférée un monomère de vinyle non ionique hydrophobe,
(C) au moins un monomère contenant un groupe terminal insaturé et une partie polyoxyalkylène,
(D) au moins un monomère réticulant,
(E) éventuellement un colloïde protecteur,
**caractérisé en ce que** la polymérisation est régulée de telle sorte que (F)
l'effet de gel se produise au moins temporairement, ce qui est obtenu **en ce que** l'ajout des monomères (A), (B) et (C) a lieu (temps d'ajout) pendant 120 minutes, de préférence 60 minutes, de manière particulièrement préférée 40 minutes, de manière tout particulièrement préférée 30 minutes,
l'ajout des monomères étant constant et ayant lieu simultanément à une vitesse d'ajout élevée des monomères,
(G) l'ajout du monomère réticulant (D) a lieu au plus tôt 10 minutes après le premier ajout des monomères (A), (B) et (C),
et
(H) des monomères associatifs de type U-S-L sont absents ou présentent au plus une concentration de 1 % en poids, de préférence de 0,1 % en poids,
les monomères associatifs de type U-S-L contenant un groupe éthyléniquement insaturé tel que méthacrylate, acrylate, allyle ou vinyle, correspondant à U, un espaceur constitué par une chaîne polyoxyalkylène de 2 à 300 unités oxyalkylène telle que polyéthylène glycol, polypropylène glycol ou polybutylène glycol, correspondant à S, et un groupe hydrophobe tel qu'un groupe alkyle et/ou aryle, qui comprend au moins 4, de préférence au moins 8 atomes de carbone, correspondant à L.

2. Polymère selon l'une quelconque des revendications précédentes, **caractérisé en ce que** le monomère de vinyle acide (A) est choisi parmi les monomères de vinyle contenant des groupes carboxyle, de manière particulièrement préférée l'acide acrylique ou l'acide méthacrylique ou leurs sels alcalins, alcalino-terreux, d'ammonium ou d'alkylammonium, de manière tout particulièrement préférée l'acide méthacrylique ou ses sels alcalins, alcalino-terreux, d'ammonium ou d'alkylammonium.

3. Polymère selon l'une quelconque des revendications précédentes, **caractérisé en ce que** le monomère de vinyle non ionique (B) est choisi dans le groupe constitué par les acrylates d'alkyle en C1 à C22 et les méthacrylates d'alkyle en C1 à C22, ainsi que leurs mélanges.

4. Polymère selon l'une quelconque des revendications précédentes, **caractérisé en ce que** le monomère (C), contenant un groupe terminal insaturé et une partie polyoxyalkylène, est choisi parmi les vinylpolyalkylène glycols ou les tensioactifs polymérisables ou leurs mélanges, de préférence est choisi parmi l'éther vinylique de 1,4-butanediol éthoxylé et propoxylé contenant 30 unités oxyéthylène et oxypropylène, les éthers d'allylpolyéthylène glycol contenant 30 unités oxyéthylène, les éthers d'allylpolyéthylène glycol contenant 20 unités oxyéthylène, les éthers de vinylpolyéthylène glycol contenant 20 unités oxyéthylène, CH₂=CHCH₂O[(CH₂CH₂O)ₙ(CH₂(CH₃)CHO)]ₘCH₃ avec m+n = 5 à 100 et n/m = 1, les éthers allylbutyliques de polyalkylène glycol contenant 25 unités oxyéthylène et 8 unités oxypropylène.

5. Polymère selon l'une quelconque des revendications précédentes, **caractérisé en ce que** le monomère réticulant (D) est choisi parmi les (méth)acrylates de polyol contenant au moins deux groupes (méth)acrylate et les esters mixtes de polyols avec de l'acide acrylique et/ou de l'acide méthacrylique.

6. Polymère selon l'une quelconque des revendications précédentes, **caractérisé en ce que** les monomère (A):(B) sont présents en des rapports en masse de 1:2,2 à 1,5:1, de préférence de 1:1,6 à 1,3:1.

7. Polymère selon l'une quelconque des revendications précédentes, **caractérisé en ce que** l'ajout du monomère réticulant débute au plus tôt après 10 minutes et soit se termine immédiatement, soit dure jusqu'à la fin de l'ajout des monomères (A), (B) et (C).

8. Polymère selon l'une quelconque des revendications précédentes, **caractérisé en ce qu'**il est choisi parmi l'exemple 7, 8 ou 12.

9. Préparation épaissie, contenant un polymère selon l'une quelconque des revendications précédentes.

10. Préparation selon la revendication 9, **caractérisée en ce que** la concentration utilisée du polymère pour l'épaississement de préparations aqueuses, de préférence aqueuses contenant des tensioactifs, est de 0,5 à 5 %, de préférence de 1 à 4 %.

11. Préparation selon la revendication 10, **caractérisée en ce que** la préparation est transparente à des pH < 6,5 et présente une viscosité d'au moins 2 000 mPa·s.

12. Préparation selon la revendication 10 ou 11, **caractérisée en ce qu'**elle consiste en un produit de santé, un produit de nettoyage, un produit ménager, un gel couche, une peinture, une encre, un agent de dispersion, un agent stabilisateur, un additif pour béton et ciment, un revêtement, un produit médical, un produit cosmétique ou un produit dermatologique.

13. Utilisation d'un polymère selon l'une quelconque des revendications 1 à 8 en tant qu'épaississant, émulsifiant, agent de dispersion ou agent texturant.

14. Procédé de fabrication d'un polymère par polymérisation en émulsion radicalaire, **caractérisé en ce que** le polymère peut être obtenu à partir de :
(A) au moins un monomère de vinyle acide ou son sel,
(B) au moins un monomère de vinyle non ionique, de manière particulièrement préférée un monomère de vinyle non ionique hydrophobe,
(C) au moins un monomère contenant un groupe terminal insaturé et une partie polyoxyalkylène,
(D) au moins un monomère réticulant et
(E) éventuellement un colloïde protecteur,
la polymérisation étant régulée de telle sorte que
(F) un effet de gel se produise au moins temporairement, ce qui est obtenu **en ce que** l'ajout des monomères (A), (B) et (C) a lieu pendant 120 minutes, de préférence 60 minutes, de manière particulièrement préférée 40 minutes, de manière tout particulièrement préférée 30 minutes,
l'ajout des monomères étant constant et ayant lieu simultanément à une vitesse d'ajout élevée des monomères, et
(G) l'ajout du monomère réticulant (D) a lieu au plus tôt 10 minutes après le premier ajout des monomères (A), (B) et (C), et
(H) des monomères associatifs de type U-S-L sont absents ou présentent au plus une concentration de 1 % en poids, de préférence de 0,1 % en poids,
les monomères associatifs de type U-S-L contenant un groupe éthyléniquement insaturé tel que méthacrylate, acrylate, allyle ou vinyle, correspondant à U, un espaceur constitué par une chaîne polyoxyalkylène de 2 à 300 unités oxyalkylène telle que polyéthylène glycol, polypropylène glycol ou polybutylène glycol, correspondant à S, et un groupe hydrophobe tel qu'un groupe alkyle et/ou aryle, qui comprend au moins 4, de préférence au moins 8 atomes de carbone, correspondant à L.

15. Procédé selon la revendication 14, **caractérisé en ce que** l'ajout du monomère réticulant débute au plus tôt après 10 minutes et soit se termine immédiatement, soit dure jusqu'à la fin de l'ajout des monomères (A), (B) et (C).
